# EUROPEAN PATENT SPECIFICATION

(11) **EP 1 508 338 B1**
(45) Date of publication and mention of the grant of the patent: **31.12.2008**
(21) Application number: 03019169.6
(22) Date of filing: 22.08.2003
(51) Int. Cl.: A61K 39/00, A61K 39/39, A61K 38/16

(54) **Protein antigens useful for cancer therapy and vaccination**
Mycobakterielle Proteinantigene für Krebstherapie und Impfung
Antigènes de protéines de mycobactèries pour la thérapie du cancer et la vaccination

(43) Date of publication of application: 23.02.2005
(73) Proprietor: Axenoll ag, 5405 Baden-Dättwil (CH)
(72) Inventor: Singh, Mahavir, Prof.Dr., 38124 Braunschweig (DE); Böhle, Andreas, Prof.Dr., 23627 Gross-Gönau (DE); Sänger, Christian, Dr., 22081 Hamburg (DE); Brandau, Sven, Dr., 23863 Kayhude (DE)
(74) Representative: Kröncke, Rolf

(56) References cited:
- WO-A-00/39301
- US-B1- 6 517 839
- SASAKI J ET AL: "Biological activity of stress protein (64 kDa) derived from Mycobacterium bovis BCG" BIOTHERAPY 1995 JAPAN, vol. 9, no. 3, 1995, pages 375-376, XP008028373 ISSN: 0914-2223
- HARMALA LISA A E ET AL: "The adjuvant effects of Mycobacterium tuberculosis heat shock protein 70 result from the rapid and prolonged activation of antigen-specific CD8+ T cells in vivo." JOURNAL OF IMMUNOLOGY, vol. 169, no. 10, 15 November 2002 (2002-11-15), pages 5622-5629, XP001176699 ISSN: 0022-1767 (ISSN print)
- MAES H ET AL: "Alteration of the immune response during cancer development and prevention by administration of a mycobacterial antigen" SCANDINAVIAN JOURNAL OF IMMUNOLOGY, vol. 41, no. 1, 1995, pages 53-64, XP008028374 ISSN: 0300-9475
- VENKATAPRASAD N ET AL: "Induction of cellular immunity to a mycobacterial antigen adsorbed on lamellar particles of lactide polymers" VACCINE, BUTTERWORTH SCIENTIFIC. GUILDFORD, GB, vol. 17, no. 15-16, 9 April 1999 (1999-04-09), pages 1814-1819, XP004165027 ISSN: 0264-410X
- DATABASE BIOSIS [Online] BIOSCIENCES INFORMATION SERVICE, PHILADELPHIA, PA, US; 1996, TKACHUK V N ET AL: "Use of BCG vaccine to prevent recurrences of superficial cancer of the bladder" XP002272854 Database accession no. PREV199699008490 & UROLOGIYA I NEFROLOGIYA, vol. 0, no. 2, 1996, pages 23-25, ISSN: 0042-1154
- SINGH M ET AL: "THE MYCOBACTERIUM-TUBERCULOSIS 38-KDA ANTIGEN OVERPRODUCTION IN ESCHERICHIA-COLI PURIFICATION AND CHARACTERIZATION" GENE (AMSTERDAM), vol. 117, no. 1, 1992, pages 53-60, XP001188903 ISSN: 0378-1119

## Description

The invention is related to the use of highly immunogenic protein antigens for vaccination and immunotherapy of cancer in humans and animals.

### Background

Prevention and treatment of cancer is still a challenge for science. Urothelial carcinoma of the bladder, for example, accounts for about 4% of all cancer death in man. The large majority of tumors (70-80%) is superficial at diagnosis and, after local surgical therapy, has a high rate of local recurrence (70%) and progression (30%). Therefore, patients require lifelong medical follow-up examinations with inspections of their bladders, and effective prophylactic treatments are needed to prevent recurrences and progression of the tumor. Clinical trials have shown that instillation of BCG (bacillus Calmette-Guerin) into the bladder is superior to topical chemotherapy and transurethral resection of the tumor to prevent recurrences and local progression especially in patients with high-risk tumors (1,2). Despite several clear advantages of BCG immunotherapy for the treatment of bladder cancer, several problems and limitations compromise its use. Although BCG is the most effective agent against superficial transitional cell carcinoma (TCC), currently there are still 30 to 40% of patients not responding to the BCG therapy (3). In the case of muscle invasive bladder cancer, BCG has not been shown to be effective at all (4). BCG's activity also appears to be strictly localized. As a living organism, BCG poses unique toxicity problems associated with its use. Although only 5% of these problems are severe, most if not all patients experience some irritable bladder symptoms (cystis) during BCG therapy (5). Roughly 40% develop hematuria and 30 % experience flu like symptoms including fever malaise, and nausea or vomiting. Actual BCG sepsis has been reported in 0.4%, including some cases, which have been fatal (6).

The reported side effects underscore the need for alternative forms of treatment, as generally desired for any types of cancers.

Several BCG derived compounds like cell wall preparations; Arabinomanans or whole BCG ghosts were investigated to serve as nonviable substitutes for therapy of bladder cancer. Tkachuk et al. Urologiya; Nefrologiya, 1996, 2, 23 - 25 noted that BCG is able to prevent recurrences of superficial bladder cancer subjected to transurethral resection in the mayority orf patients. But none of these substances could be successfully used in preclinical therapy studies.

PstS1 is a phosphate transport protein located in the cell wall of *Mycobacterium tuberculosis, M.bovis* and *M.bovis* BCG. It is a glycosylated lipoprotein which is also secreted in the extracellular culture supernatant (7,8). Moreover, several studies have shown that PstS1 represents one of the most immunogenic antigens with potential applications in diagnosis and vaccine development for tuberculosis (9-20). It is involved in active uptake and transport of inorganic phoshpate across the membrane. This is one of the protein required for binding protein mediated phosphate transport in tuberculosis. The sequence of PstS1 is for example provided in EP 519 218, its Genebank accession number is e.g. M30046. In EP 926 156 the use of expression vectors containing a nucleic acid sequence encoding PstS1 for the treatment of neoplasm is disclosed. However, it is indicated therein that the protein itself does not prevent tumor formation. This is in accordance with the common knowledge that a different way of processing of endogenous and exogenous antigens and their presentation exists. Thus, exogenous administration normally does not elicit the same immune response as the endogenous expression, see e.g. (21).

In view of the above, it is an object of the invention to find a composition for the prophylaxis and for the treatment of vaious types of cancer and especially for the treatment of urothelial carcinoma of the bladder.

### Summary of the Invention

The inventors found a remarkable anti-tumor potential with recombinant mycobacterial PstS1, especially, but not necessarily, in the context of an unspecific immunstimulation with DL-lactide particles (L-particle).

PstS1, similar to BCG, is able to induce proliferation of lymphocytes, IFN-γ release of PBMC and cell mediated cytotoxicity against T24 bladder carcinoma cells of PBMCs *in vitro.* Further dosage kinetics had been carried out with PstS1 in the readout systems mentioned above. These findings led to *in vivo* therapy experiments in a murine orthotopic bladder cancer model which revealed that mice, preferably prevaccinated with L-particles and treated with PstS1 intravesically revealed a significant higher survival ratio than control animals.

One aspect of the invention is the use of a wild-type or recombinant mycobacterial PStS1 protein or a homologous protein or a synthetic equivalent or a therapeutically effective part of one of these as defined below, for the preparation of a vaccine or a therapeutical composition for the treatment of cancer.

In another aspect, the invention relates to the use of PstS1 in combination with L-particles.

As the invention provides for an immunostimulation, according to the present invention, a treatment and/or vaccination for any types of cancers is possible, e.g. cancers of breast, lung, bladder, melanoma, or other.

### Detailed description of the invention

As used herein, the term "PstS1 protein" is defined by the complete amino acid sequence corresponding to the GeneBank accession number M30046. Included in this definition are variants of the proteins showing at least 60%, e.g. at least 70%, at least 80%, preferably at least 90%, e.g. at least 95% or at least 99% homology to the above GeneBank accession numbers. These variants of the above proteins can be present in nature or can originate from non-natural sources. Natural sources can be proteins of different species, etc. Non-natural sources can be for example site-directed mutagenesis or random mutagenesis, the latter of which can ber achieved for example by chemicals or by the use of ionizing radiation. The amino acid sequences may inlcude substitutions, deletions, insertions or combinations of these alternations. The resulting proteins can be biologically active, partial biologically active or biologically inactive as long as they are immunologically effective.

The term "protein fragments" refers to all fragments of the corresponding proteins, i.e. PstS1. Preferably these fragments are at least 8 amino acids long. These protein fragments share at least 70%, preferably 80%, preferably 90%, e.g. 95% or 99% homology to the natural amino acid sequences as disclosed in GeneBank. Percentage of homology is calculated on the basis of the length of the protein fragment not on the basis of the length of the protein. Thus, a protein fragment with a sequence length of 20 amino acids which includes 4 amino acids different from the corresponding sequence of the published protein is a 80% homologue fragment of the published protein.

To determine the homology between amino acid sequences several computer software packages are known in the art.

The protein may be isolated from natural sources or may be prepared by recombinant technologies or chemical synthesis protocols known to the skilled person. Also purification thereof may be performed by commonly known techniques.

The amino acid sequence may be modified chemically or post-translationally. For example, the amino acid sequence may consist of both D- and L-amino acids, and combinations thereof. These sequences may also comprise modified and/or unusual amino acids. Further, at least one of the amino acids in the respective amino acid sequence may be post-translationally modified, e.g. by phosphate or sulphate-modifications, glycosilation, amidation, deamidation, oxidized or amidated amino acid side chains, etc. These substances are also known as equivalents of the respective proteins.

The term PstS1 as used herein encompass the protein itself as well as the fragments, equivalents and homologues as defined above.

The term "vaccination" as used herein means that the object to be vaccinated receives an effective amount of the vaccine to elicit an immune response in order to establish protective immunization.

Vaccination may be obtained by administering the vaccine in advance, that means to elicit a protective immunization, which is also sometimes referred to as preventive vaccination. Vaccination may also be effected as a therapeutic measure. In particular in cancer therapie vaccines are adminstered after or in combination with other therapeutic measures after tumor identification. This type of vaccination is also known as therapeutic vaccination.

Moreover, in case two different types of compounds are used as vaccine, these compounds may be administered simultaneously, separately or sequentially. In addition, a first compound may be given, which is also called a prevaccination. Afterwards the second compound is administered. For example, the second compound may be given after some hours or days later, e.g. 1, 2, 3 etc days later.

The efficacy of the proteins, equivalents, fragments and homologues thereof as a vaccine or a therapeutical composition is e.g. expressed in the survival rate of treated mice as exemplified below. That means an effective substance according to the present invention is able to increase the survival rate when compared to a control group which did not receive the vaccine.

The PstS1 antigen protein according to this invention is a *Mycobacterium tuberculosis* antigen of approximately 38 kDa (Ag38) or a homologous protein of approx. 33 to 38 kDa, each wild-type or recombinant (7, 8, 12, 14). As regards the known 38 kDa antigen of *Mycobacterium tuberculosis* and homologues thereof reference can be made for example to EP-A-92 108 254.1 and references cited therein. Within the scope of this invention equivalents or therapeutically effective fragments thereof and of the Hsp16 protein are included. A "therapeutically effective fragment" is a fragment of the respective protein that is - like the PstS1 - effective in a preparation for a vaccine or a therapeutical composition for the treatment of cancer as disclosed in this invention.

One preferred embodiment is the use for the preparation of a vaccine or a therapeutical composition for the treatment of an urothelial carcinoma of the bladder or a mama carcinoma.

The vaccine or the therapeutical composition according to the invention may also contain pharmaceutically acceptable carriers, adjuvants, additional pharmaceutical ingredients, drugs and/or additives. The skilled practitioner will make use of these options in case it seems appropriate.

Further it is possible that the vaccine or the preparation or a kit of preparations contains DL-lactide particles (L-particles) and/or dendritic cells, preferably derived from blood monocytes.

Preferably each protein is present at a concentration from 0.1 to 100 µg/ml.

The invention also includes a method of treatment of an individual suffering from carcinoma, comprising the step of administering at least once a wild-type or recombinant mycobacterial PStS1 protein or a homologous protein or an equivalent or a therapeutically effective fragment of one of these and/or a wild-type or recombinant Hsp 16 protein or a homologous protein or an equivalent or a therapeutically effective fragment of one of these to the respective individual.

Prior to the administration of PstS1 the method preferably comprises a prevaccination of said individual with an unspecific immunostimulant, which may preferably be a composition comprising DL-lactide particles (L-particles) also including the possibility of prevaccinating with DL-lactide particles alone. Again the proteins are preferably used in concentrations ranging from 0.1 to 100 µg/ml. Alternatively, the unspecific immunostimulant, may be given simultaneously with the protein(s) according to the present invention. The schedule for vaccination may depend on the kind of therapy or prophylaxis. That is, the vaccination may be a preventive or a therapeutic vaccination.

Where the carcinoma is an urothelial carcinoma of the bladder, the administration can be at least intravesically into the bladder. Other pathways are within the scope of the invention. Explicitly, a systemic administration is possible, since both antigens are non-toxic, when applied in moderate dosages. Of course, the administration pathway also depends on the type of cancer to be treated. Adequate pathways and galenics can be chosen by the practitioner in the field.

For example, in case of urothelial carcinoma of the bladder, the protein (PstS1) may be administered alone with or without prevaccination with e.g. L-particles.

In case of other administration pathways, a prevaccination with L-particles may be performed before giving PstS1, or, alternatively, a composition of L-particles and the protein(s) is administered.

An appropriate regimen can provide repeated treatments, wherein the method steps as cited above are at least once repeated within a period of 1 - 28 days.

The invention will now be described in detail with reference to the figures and examples below.

### Brief Description of the figures

- Fig. 1: shows that PstS1 is able to stimulate PBMCs *in vitro;*
- Fig. 2: shows that PstS1 stimulation of PBMCs is dose dependent;
- Fig.3: shows the prolonged survival of L-particle stimulated C57/BL6 mice after intravesical PstS1 therapy;
- Fig.4: shows the prolonged survival of L-particle stimulated C57/BL6 mice after intravesical PstS1 therapy.

### METHODS

*In-vitro* Activation of Peripheral Blood Mononuclear Cells (PBMC)

### Cell Culture

The human bladder tumor cell line T-24 was cultured at 37°C and 5% CO₂ in RPMI 1640 containing 10% FCS, 1% glutamine, 100 U/ml penicillin and 100 µg/ml streptomycin. The murine bladder tumor cell line MB-49 was cultured in DMEM containing 10% FCS, 1% L-glutamine, 100 U/ml penicillin and 100 µg/ml streptomycin.

### Generation of BAK Cells and PstS1 activated PBMCs

MNCs from heparinized blood of healthy human donors were obtained by Biocoll Separating Solution (Biochrom, Berlin/Germany) centrifugation and adjusted to a concentration of 1X10⁶/ml in RPMI-1640 medium (PAA Laboratories, Linz/Austria) containing 5% of human serum, 100 U/ml penicillin and 100 µg/ml streptomycin. Recombinant PstS1 (38kDa, soluble, Lionex GmbH, Braunschweig) or Hsp16 (16kDa, soluble, Lionex GmbH, Braunschweig) in concentrations from 0,1 µg/ml - 100µg/ml, BCG (Connaught substrain, Immucyst, 4X10⁴ cfu/ml), or PBS (100 µl) was added and the cells were cultured for 7 days in 6-well microtiter plates at 37°C and 5% CO₂.

### Chromium Release Assay

Cytotoxicity was determined in a standard 4-hour chromium release assay. Target cells were labelled with Na₂⁵¹CrO4 for 1h at 37°C, washed and resuspended at 5X10⁴ cells/ml. Effector cells were added to a total of 100µl of target cells at an effector: target ratio of 40:1. The radioactive content of the supernatant was determined in a gamma-counter (Berthold, Wildbad/Germany). The specific lysis was determined according to the formula: spec. lysis (%) = 100 X (Exp - Spo)/ (Max - Spo) where Exp is the experimental release, Spo the spontaneous release and Max the maximum release. All assays were performed in triplicate with different donors.

### IFN-γ Detection

Supernatants of PstS1, BCG and PBS stimulated PBMCs were recovered at day 7 and examined for the presence of IFN-γ. Each experiment was carried out several times with different donors. Detection was performed with an ELISA-Kit from Bioscience (San Diego, USA).

### Proliferation Assay

PBMCs were stimulated for 7 days with PstS1, BCG or PBS. 2x10⁴ / well of the stimulated PBMCs were cultured in a microwell plate together with 1µCi ³H⁻Thymidin (five wells per sample). DNA was harvested on filter membranes and thymidin incorporation was measured by liquid scintillation counting (counter: LKB Wallace 1205 beta-plate).

### L-particle Preparation

L-particles (10mg/ml) (Lionex GmbH) were diluted 4:1 with PBS and the pH was adjusted to 7.0 with NaOH.

### L-particle Vaccination and Immunotherapy in C57BL/6 Mice

6 to 8 weeks old female C57BL/6 mice were purchased from Charles-River Laboratories. To test efficiency of PstS1 therapy in C57/BL6 mice a published syngeneic orthotopic bladder cancer model was used (10). Mice (trial I: 15 animals per group; trial II: 11 animals per group) were subcutaneously vaccinated with 250µg L-particles or 100µl of PBS. Ten days later the mice were anesthetized by intraperitoneal treatment with Pentobarbital (0.067 mg/g). After insertion of a 24-gauge Teflon intravenous catheter (Insyte-W, Becton Dickinson, Germany) transurethrally into the bladder, electro coagulation was performed with a guide wire. Thereafter 6x10⁴ MB-49 cells were intravesically instilled into the bladder. Intravesical immunotherapy with 100 µg PstS1 in 100 µl PBS was performed on days 1, 8, 15 and 22 after tumor implantation. Control groups were instilled with PBS (100 µl) alone. The viability status of the mice was checked daily. Surviving mice were sacrificed on day 70. Survival of mice was compared using Kaplan-Meier analysis and log-rank test.

### Splenocyte Proliferation Assay

The surviving mice of the second therapy experiment were sacrificed on day 120 after tumor instillation. Splenocytes of three mice per group were washed out with RPMI. After washing the remaining cells with PBS erythrocytes were lysed with H₂O. Afterwards 2x10⁴ splenocytes per well were cultured in a microwell plate together with 10 µg / ml PstS1 for a period of 2 days (five wells per sample). Then 1µCi ³H⁻Thymidin was added and cells were incubated for 15 h at 37°C and 5% CO₂. Finally DNA was harvested on filter membranes and thymidin incorporation was measured by liquid scintillation counting (counter: LKB Wallace 1205 beta-plate).

### α-PstS1-ELISA from murine peripheral blood

120 days after tumor implantation peripheral blood of all mice was collected and centrifuged down at 2000 rpm in a minifuge. The serum was used for the determination of IgG-antibodies to PstS1 using a kit developed by Lionex GmbH.

### EXAMPLES

To investigate the immune response against PstS1 and Hsp16 a splenocyte-restimulation assay was carried out and the presence of anti-PstS1 or anti-Hsp16 antibodies in the peripheral blood of the mice was determined.

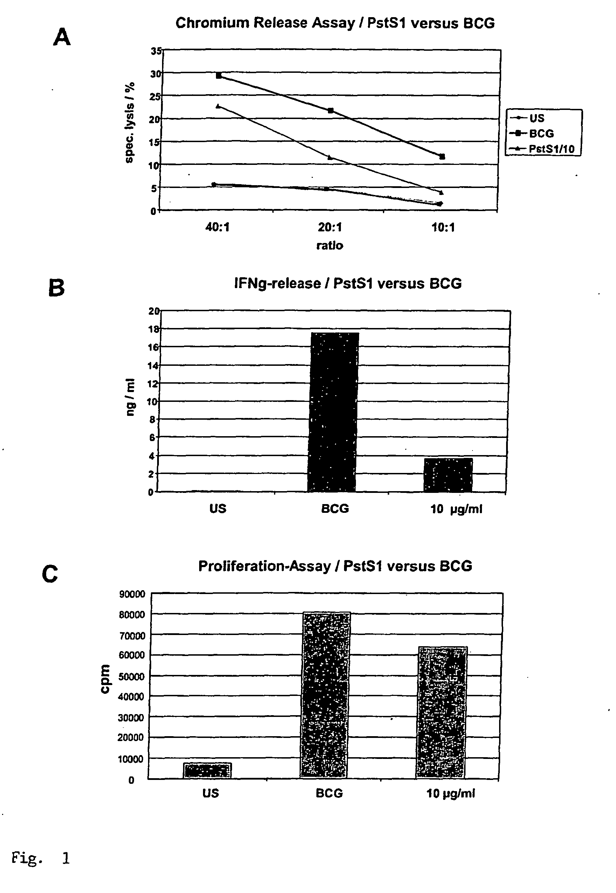

PstS1 is able to stimulate PBMCs in vitro To determine the ability of PstS1 to stimulate PBMCs the cells were incubated with 10 µg/ml PstS1.IFN-γ production, lymphocyte proliferation, and cytotoxicity against T24 bladder tumor cells was chosen as a readout system. The results show clearly, that compared with the negative control, PstS1 is able to stimulate IFS-γ production, to induce lymphocyte proliferation and cytotoxicity of PBMCs (Fig.1). Results are shown for PstS1. While PstS1-mediated cytotoxicity and proliferation were comparable with the effects mediated by BCG, PstS1 was less potent than BCG in inducing IFN-γ from PBMCs. Nevertheless, in every donor tested substantial amounts of IFN-γ (1ng to 14ng/ml) were secreted after PstS1 stimulation. As expected the magnitude of the immune-stimulatory capacity was variable between different donors. However, PstS1 reproducibly induced IFN-γ-production, lymphocyte proliferation and cytotoxicity as compared to un-stimulated controls.

Fig. 1 shows that PstS1 is able to stimulate PBMCs *in vitro*. PBMCs were stimulated for 7 days with PstS1 (10µg/ml), BCG (4X10⁴ cfu/ml) or PBS (100 µl). **A** 4-hour chromium release assay against T-24 bladder tumor cells with Effector-target cell ratios of 40:1, 20:1 and 10:1 **B** IFN-γ release was determined by ELISA from supernatants of the stimulated PBMCs **C** T-cell proliferation was measured by overnight ³H-Thymidin incorporation assay (1 µCi/2x10⁴ cells).

### PstS1 stimulation of PBMCs is dose dependent

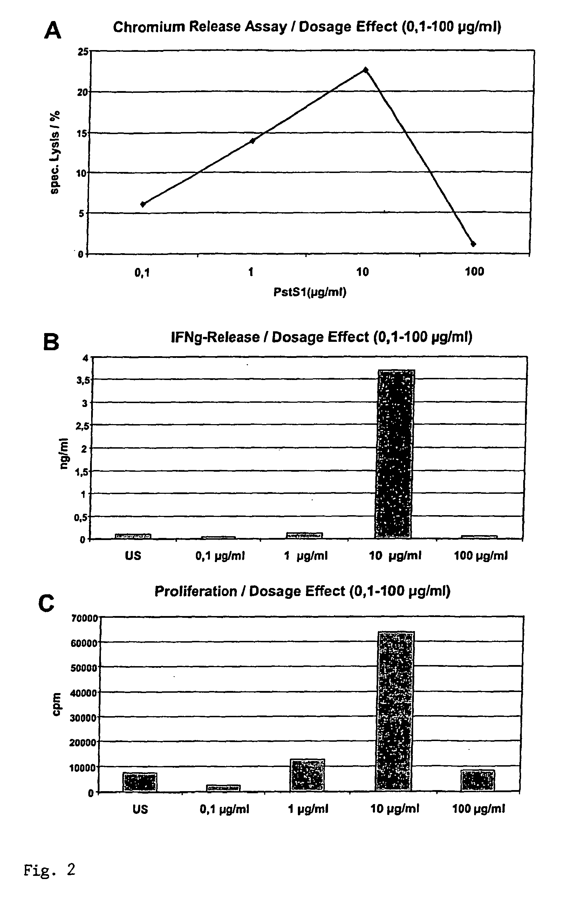

Cytotoxicity, lymphocyte proliferation and IFN-γ release were measured from PBMCs stimulated for 7 days with PstS1 doses of 0,1µg/ml, 1 µg/ml, 10 µg/ml and 100µg/ml. In all three readout systems a dose of 10µg/ml was optimal for the respective immune-stimulation (Fig. 2). PstS1 stimulation of PBMCs showed a typical optimal dose response curve with a peak at about 10 µg/ml.

Fig. 2 shows that PstS1 stimulation of PBMCs is dose dependent. PBMCs were stimulated for 7 days with different PstS1 concentrations in a range from 0,1 µg/ml to 100 µg/ml. **A**: 4-hour chromium release assay against T-24 bladder tumor cells. Effector-target cell ratio of 40:1. **B**: IFN-γ release was determined by ELISA from supernatants of the stimulated PBMCs. **C:** T-cell proliferation was measured by overnight ³H-Thymidin incorporation assay (1 µCi/2x10⁴ cells).

### Intravesical Immunotherapy with PstS1 after pre-stimulation with L-particles

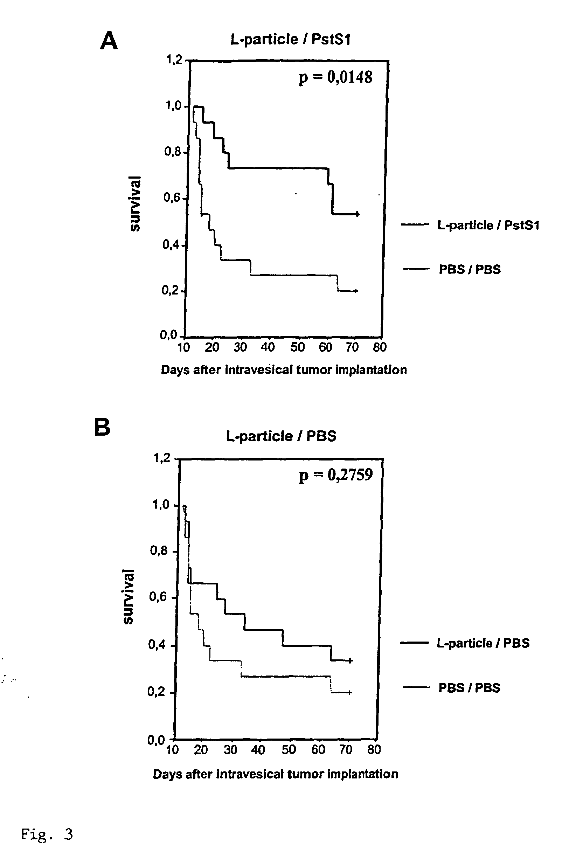

To determine the therapeutical potential of PstS1, the survival of L-particle prevaccinated tumor bearing C57/BL6 mice was monitored, which were treated intravesically four times in weekly intervals with PstS1. PBS pre-vaccinated and treated mice served as tumor take control (control group). In the therapeutical experiment depicted in Fig.3A we could show that, in contrast to the control group, L-particle-prevaccinated, PstS1-treated mice revealed a significantly prolonged survival (p = 0,0148). In contrast, mice pre-vaccinated with L-particles but treated with PBS showed no significantly prolonged survival compared with the control group (p = 0,2759).

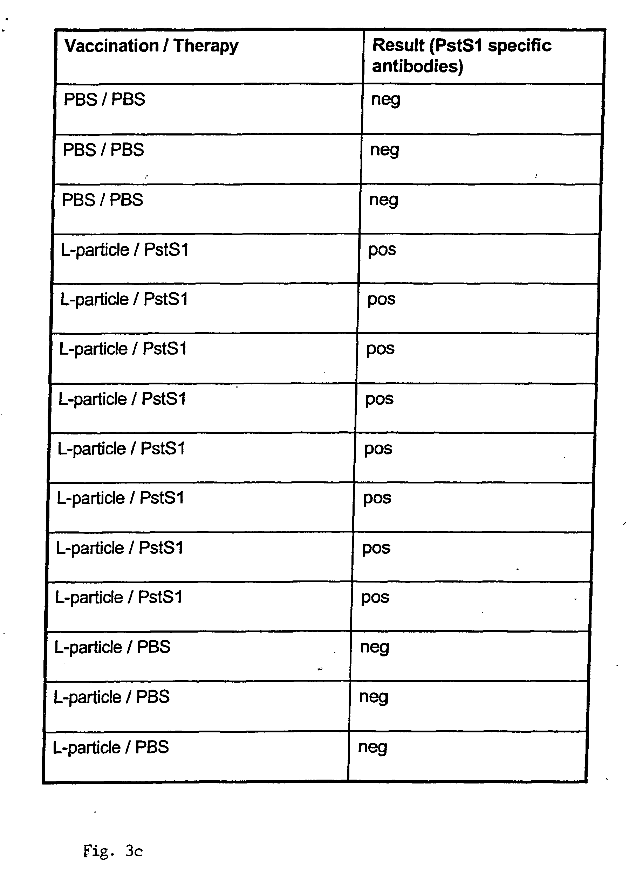

Fig. 3 shows the prolonged survival of L-particle stimulated C57/BL6 mice after intravesical PstS1 therapy. ***Trial-1,* A**: L-particle prevaccinated, tumor bearing C57/BL6 mice after four weekly PstS1 instillations (P = 0,0148). L-Particle prevaccination/PstS1 therapy = black graph; PBS prevaccination/PBS therapy = grey graph. **B**: Tumor bearing mice vaccinated with L-particles but instilled four times with PBS (P = 0,2759). L-Particle prevaccination/PBS therapy = black graph; PBS prevaccination/PBS therapy = grey graph. Significance was calculated with Kaplan Meier analysis and log-rank test. **C**: 100 days after tumor implantation, humoral response in mice was studied by measuring the specific IgG antibodies generated by PstS1 treatment.

### Intravesical Immunotherapy with PstS1 after pre-stimulation with L-Particles (2. Trial)

In a second trial, mice were pre-vaccinated with PBS and L-particles, respectively, and afterwards intravesically inoculated. Both strategies led to a significantly higher number of surviving mice (chi-square) further substantiating the therapeutic potential of intravesical PstS1.

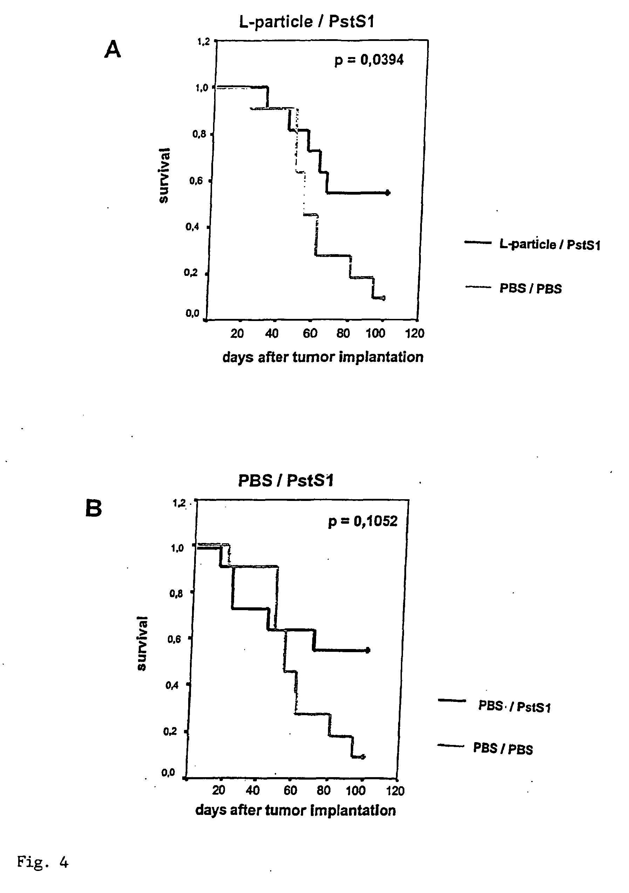

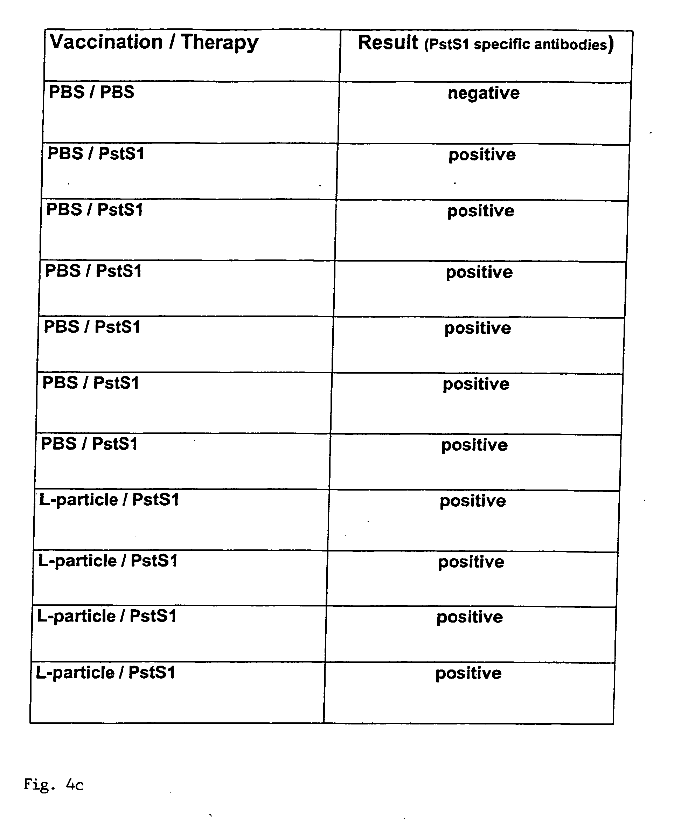

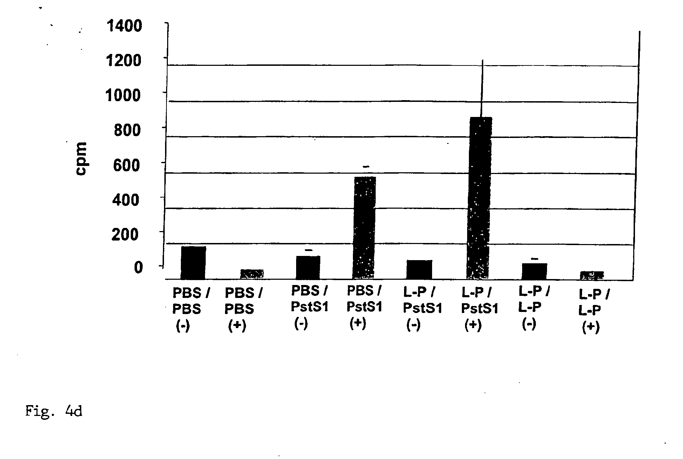

Fig. 4 shows the prolonged survival of L-particle stimulated C57/BL6 mice after intravesical PstS1 therapy.*Trial II* **A,** L-particle pre-vaccinated, tumor bearing C57/BL6 mice after four weekly PstS1 instillations (P = 0,0394). L-Particle prevaccination/PstS1 therapy = black graph; PBS pre-vaccination/PBS therapy = grey graph. **B**, Tumor bearing mice vaccinated with PBS and instilled four times with PstS1 (P = 0,1052).
**C**, 120 days after tumor implantation sera of the mice were analyzed by an ELISA for detecting antibodies specific to PstS1.
**D**, 120 days after tumor implantation, the isolated Splenocytes of surviving mice (three animals per group, one animal of control group) were re-stimulated with PstS1 (10 µg/ml) for two days and proliferation was analyzed by ³H-Thymidin incorporation. Compared to PBS/PBS control and L-particle/L-particle mice, the splenocytes of PBS/PstS1, L-particle/PstS1 and L-BSA/PstS1 groups showed an increased proliferation rate. Black columns = re-stimulation control with PBS. Grey columns = re-stimulation with PstS1. In case of L-particle/PstS1 and L-BSA/PstS1 groups, one of the three animals showed no enhanced splenocyte proliferation. These data are not shown in the diagram.

### Splenocyte Proliferation

The fact that, after two days of incubation with PstS1, proliferation of splenocytes from PstS1 treated mice could be observed, is a strong indication for the presence of PstS1 specific memory T-cells (Fig. 4D).

### Humoral response

We also studied the serological response to PstS1 treatment in mice. All PstS1 treated mice showed serum IgG antibodies to PstS1. None of the mice from control group showed any antibodies to PstS1 (Fig. 4C).

Both results show the interaction between intravesically administered PstS1 and components of the host immune system.

### CONCLUSIONS

Taken together, these findings show that in the orthotopic mouse model and in the context of pre-vaccination with L-particles, PstS1 shows a distinct therapeutic effect leading to long term survival of mice, whereas L-particle immunisation alone is not sufficient for a successful therapy. Treatment with PstS1 without pre-stimulation with L-Particles results in a considerable higher number of surviving animals and demonstrates that this mycobacterial antigen alone also shows considerable antitumor potential.

### References cited

1) Lamm DL, Blumenstein BA, Crawford ED, Montie JE, Scardino P, Grossman HB, Stanisic TH, Smith JA Jr, Sullivan J, Sarosdy MF, et al., N Engl J Med. 1991 Oct 24;325(17):1205-9.
2) Herr HW, Schwalb DM, Zhang ZF, Sogani PC, Fair WR, Whitmore WF Jr, Oettgen HF. J Clin Oncol. 1995 Jun;13(6):1404-8.
3) Herr HW, Badalament RA, Amato DA, Laudone VP, Fair WR, Whitmore WF Jr., J Urol. 1989 Jan;141(1):22-9.
4) Cymes M et al., J Urol 147, 273A, 1992
5) Lamm DL., Urol Clin North Am. 1992 Aug;19(3):573-80. Review. 6) Steg A et al., Eur Urol, 21, 35, 1992
6) Lamm D.L., et al., EORTC Genito-Urinary Group Monograph 6 : BCG in superficial bladder cancer, Debruyne, F M J, Denis, L, v.d.Meijden A P M, pp. 335-355, Liss, New York, 1989
7) Espitia C, Mancilla R., Clin Exp Immunol. 1989 Sep;77(3):378-83.
8) Young DB, Garbe TR., Res Microbiol. 1991 Jan;142(1):55-65.
9) Ivanyi J et al., Tuberculosis : Pathogenesis, Protection and Control (Ed. Bloom, B). ASM Press, Washington, 1994, pp 437
10) Gunther JH, Jurczok A, Wulf T, Brandau S, Deinert I, Jocham D, Bohle A., Cancer Res. 1999 Jun 15;59(12):2834-7.
11) Harris D P, Vordermeier H-M, Singh M, Moreno C, Jurcevic, S. and Ivanyi J (1995). Eur. J. Immunol. 25, 3173-3179
12) Wilkinson R J , Haslov K, Rappuoli R, Giovannoni F, Narayanan P N, Desai C R, Vordermeier M, Pausen J, Pasvol G, Ivanyi J and Singh M (1997). J. Clin. Microbiol. 35, 553-557.
13) Zhu X, Venkataprasad N, Thangaraj H, Hill M, Singh M, Ivanyi J and Vordermeier M (1997). J. Immunol. 158, 5921-5926.
14) Da Fonseca D P A J, Joosten D, Van der Zee R, Jue D I, Singh M, Vordermeier H M, Snippe H and Verheul A F M (1998) Infect. Immun. 66, 3190-3197.
15) Tanghe A, Lefévre, P, Denis O, D'Souza S, Braibant M, Lozes E, Singh M, Montogomery D, Content J and Huygen K (1999) J. Immunol. 162, 1113-1119.
16) Venkataprasad N, Coombes, A G A, Singh M, Rohde M, Wilkinson K, Hudecz F, Davies S S and Vordermeier H M (1999) Vaccine 17, 1814-1819
17) Wilkinson K A, Katoch K, Sengupta U, Singh M, Sarin K K, Ivanyi J and Wilkinson R J (1999) J. Infect. Dis. 179, 1034-1037.
18) Dieli F, Friscia G, Di Sano C, Ivanyi J, Singh M, Spallek R, Sireci G, Titone I, and Salerno A (1999) J. Inf. Dis. 180, 225-228.
19) da Fonseca DP, Frerichs J, Singh M, Snippe H, Verheul AF Vaccine. 2000 Aug 15;19(1):122-31.39:
20) Imaz MS, Comini MA, Zerbini E, Sequeira MD, Spoletti MJ, Etchart AA, Pagano HJ, Bonifasich E, Diaz N, Claus JD, Singh M Int J Tuberc Lung Dis. 2001 Nov;5(11):1036-43.
21) Beltz GT, Carbone FR, Heath WR,Crit Rev Immunol, 2002, 22 (5-6), 439-448

## Claims

1. Use of wild-type or recombinant mycobacterial PStS1 protein in the preparation of a vaccine or a therapeutical composition for the treatment of cancer.

2. The use of claim 1, where the cancer to be treated is an urothelial carcinoma of the bladder, a mamma carcinoma or lung cancer.

3. The use according to claim 1 or 2, wherein the vaccine or the therapeutical composition contains pharmaceutically acceptable carriers, adjuvants, additional pharmaceutical ingredients, drugs and/or additives.

4. The use according to one of claims 1 to 3, wherein the vaccine further contains an unspecific immunostimulant in a single composition or separately.

5. The use according to claim 4, wherein the vaccine contains DL-lactide particles as an unspecific immunostimulant.

6. The use according to one of claims 1 to 5, wherein said protein is present at a concentration from 0.1 µg/ml to 100 µg/ml.

7. The use according to claim 4 or 5 wherein the prevaccination is with said unspecific immunostimulant following by at least once the use of a wild-type or recombinant mycobacterial PstS1 protein.

8. The use according to claim 2 for the treatment of urothelial carcinoma of the bladder wherein the therapeutical composition is adapted to be administered intravesically into the bladder.

9. The use according to any one of the preceding claims, wherein the therapeutical composition is adapted to be administered at least twice within a period of 1 to 28 days.

## Patentansprüche

1. Verwendung von Wildtyp oder rekombinanten mykrobakteriellen PStS1 Protein zur Herstellung eines Vakzines oder einer therapeutischen Zusammensetzung zur Behandlung von Krebs.

2. Verwendung nach Anspruch 1, wobei der zu behandelnde Krebs ein urotheliales Karzinom der Blase, ein Mammakarzinom oder Lungenkrebs ist.

3. Verwendung nach Anspruch 1 oder 2, wobei das Vakzin oder die therapeutische Zusammensetzung pharmazeutisch annehmbare Träger, Adjuvanzien, zusätzliche pharmazeutische Inhaltstoffe, Arzneimittel und/oder Additive enthält.

4. Verwendung nach einem der Ansprüche 1 bis 3, wobei das Vakzin weiterhin ein unspezifisches Immunstimulanz in einer einzigen Zusammensetzung oder getrennt, enthält.

5. Verwendung nach Anspruch 4, wobei das Vakzin DL-Laktid Partikel als ein spezifisches Immunstimulanz enthält.

6. Verwendung nach einem der Ansprüche 1 bis 5, wobei das Protein in einer Konzentration von 0,1 µg/ml bis 100 µg/ml vorliegt.

7. Verwendung nach Anspruch 4 oder 5, wobei die Prävakzinierung mit dem unspezifischen Immunstimulanz erfolgt, gefolgt von mindestens der einmaligen Verwendung eines Wildtyp oder rekombinantem mykrobakteriellen PStS1 Proteins.

8. Verwendung nach Anspruch 2, zur Behandlung von urothelialem Karzinom der Blase, wobei die therapeutische Zusammensetzung an die intravesikale Verabreichung in die Blase angepasst ist.

9. Verwendung nach einem der vorherigen Ansprüche, wobei die therapeutische Zusammensetzung zur mindestens zweimaligen Verabreichung innerhalb eines Zeitraums von 1 bis 28 Tagen angepasst ist.

## Revendications

1. Utilisation d'une protéine PStS1 mycobactérienne recombinante ou de type sauvage dans la préparation d'un vaccin ou d'une composition thérapeutique pour le traitement du cancer.

2. Utilisation selon la revendication 1, dans laquelle le cancer à traiter est un cancer urothélial de la vessie, un cancer du sein ou un cancer du poumon.

3. Utilisation selon les revendications 1 ou 2, dans laquelle le vaccin ou la composition thérapeutique contient des excipients, adjuvants, ingrédients pharmaceutiques supplémentaires, médicaments et/ou additifs pharmaceutiquement acceptables.

4. Utilisation selon l'une quelconque des revendications 1 à 3, dans laquelle le vaccin contient en outre un immunostimulant non spécifique dans une seule composition ou séparément.

5. Utilisation selon la revendication 4, dans laquelle le vaccin contient des particules de DL-lactide comme immunostimulant non spécifique.

6. Utilisation selon l'une des revendications 1 à 5, dans laquelle ladite protéine est présente à une concentration de 0,1 µg/ml à 100 µg/ml.

7. Utilisation selon la revendication 4 ou 5, dans laquelle la pré-vaccination se fait avec ledit immunostimulant non spécifique, suivie au moins une fois par l'utilisation d'une protéine PstS1 mycobactérienne recombinante ou de type sauvage.

8. Utilisation selon la revendication 2, pour le traitement du cancer urothélial de la vessie, dans laquelle la composition thérapeutique est adaptée pour être administrée par voie intra-vésicale dans la vessie.

9. Utilisation selon l'une quelconque des revendications précédentes, dans laquelle la composition thérapeutique est adaptée pour être administrée au moins deux fois sur une durée de 1 à 28 jours.
